Europäisches Patentamt

(19) European Patent Office

Office européen des brevets

(11) Publication number: 0 265 411
A2

(12) # EUROPEAN PATENT APPLICATION

(21) Application number: 87870146.5

(22) Date of filing: 21.10.87

(51) Int. Cl.⁴: **G 01 N 33/531**
G 01 N 33/50, G 01 N 33/554,
C 07 K 3/08

(30) Priority: 22.10.86 US 921305

(43) Date of publication of application:
27.04.88 Bulletin 88/17

(84) Designated Contracting States:
AT BE CH DE ES FR GB GR IT LI LU NL SE

(71) Applicant: **Monsanto Company**
**Patent Department 800 North Lindbergh Boulevard**
**St. Louis Missouri 63167 (US)**

(72) Inventor: **Flaherty, Dennis Keith**
**270 Oak Pass Court**
**Ballwin Missouri 63011 (US)**

**Winzenburger, Peggy Ann**
**2516 Hillsboro Valley Park Road**
**High Ridge Missouri 63049 (US)**

(74) Representative: **Lunt, John Cooper et al**
**Monsanto Europe S.A. Patent Department Avenue de**
**Tervuren 270-272 Letter Box No 1**
**B-1150 Brussels (BE)**

(54) In vitro method to detect allergic reactions to low molecular weight chemicals.

(57) This invention relates to the use of IgE sensitized basophiles and mast cells in in vitro tests to determine histamine release when challenged with small molecular weight chemical haptens conjugated to protein, e.g., human serum albumin.

## Description

### IN VITRO METHOD TO DETECT ALLERGIC REACTIONS TO LOW MOLECULAR WEIGHT CHEMICALS

Field of the Invention

The present invention relates to the field of methods and means to detect allergic reactions in mammalian subjects by in vitro detection of histamine released from basophiles and/or mast cells.

Background of the Invention

It is often difficult to distinguish between irritation and allergic sensitization to low molecular weight chemicals.

Allergic reactions mediated by the IgE class of antibodies are exquisitely simple. The circulating antibody binds to two cell types in the body. Basophiles are a subpopulation of white blood cells which circulate within the blood. Mast cells are fixed in the respiratory and intestinal tracts. Basophiles and mast cells are the only cells in the body which contain histamine. The specific interaction of an allergen with the cell-bound IgE initiates the release of histamine and other mediators.

Histamine acts on the body in two ways. If histamine is released from mast cells in the lung, histamine contracts smooth muscle. The contraction of smooth muscle narrows the air passages. This results in the classical symptoms of asthma. Conversely, release of histamine from basophiles in the small blood vessels in the skin increases the permeability of the blood vessels. The increased permeability allows fluid from the blood to accumulate in the skin. Localization of fluid results in a hive which is characteristic of an allergic reaction.

The diagnosis of allergic sensitivity depends on (1) clinical history, and (2) positive skin test.

The diagnosis of allergic sensitivity to small molecular weight chemicals is difficult. The presence of circulating IgE directed toward small molecular weight chemicals does not correlate with symptoms. Moreover, skin test reagents cannot be easily generated to small molecular weight compounds.

Moreover, some individuals are hypersensitive to skin tests for allergic reaction, extreme cases of which can cause anaphylactic shock, sometimes resulting in death.

Therefore, in vitro tests have been developed in order to detect and document allergic reactions by measuring histamine released from basophile and/or mast cells.

Various in vitro methods have been developed by workers in the art based on a variety of parameters. For example, J. Benveniste (Clin. Allergy, 1981, Vol. II pp. 1-11) has described a basophile degranulation test based on basophile staining process which is said to correlate with histamine release and radioallergosorbent test. R. P. Siraganian has developed an automated fluorometric technique for the extraction and assay of histamine released from washed leucocytes and whole blood. (Manual of Clinical Immunology, N. Rose Ed., American Society for Microbiology, Washington, D.C. 1976).

C. R. Kinsolving described a method for determining histamine release from mast cells under the influence of an antigen by measuring the amount of fluorescent acridones absorbed by intact mast cells. The amount of acridone absorbed is directly proportional to amount of histamine released from the cell.

S. Skov et al (Allergy 34: 261-263, 1979) have developed another method based on in vitro incorporation of radioactively-labelled histamine in the basophile cells of the subject, wherein the release of labelled histamine is determined after provocation with suspected allergen.

The above procedures are exemplary of methods used to detect histamine release and susceptibility of humans and laboratory animals to allergic reactions.

It has not been described in the prior art to our knowledge to utilize an in vitro system as described hereinbelow to detect IgE or IgG antibodymediated allergic reactions to low molecular weight chemicals, drugs, etc.

Summary of the Invention

The present invention provides an in vitro method for determining the susceptibility of mammalian subjects to allergic reaction from a hapten which comprises:

(a) Preparing a sample of a conjugate of said hapten with protein;
(b) Providing a sample of basophile or mast cells to which is bound IgE;
(c) Contacting said hapten: protein conjugate with said basophile or mast cells bound with IgE and
(d) Measuring the amount of histamine released from said basophile and mast cells.

In the present method the basophile and are present in the plasma of peripheral blood whereas mast cells are present in tissues of all mammals, human and laboratory animals.

The hapten: protein conjugate itself is a novel composition of matter and is formed from low molecular weight chemicals, drugs, etc., coupled to a protein, e.g., human serum albumin (HSA), etc. It is essential in this in vitro test that the hapten have a molecular weight of less than about 10,000, and is uniquely suitable for compounds having a molecular weight of less than 5,000.

Haptens of industrial and agricultural chemicals are targets of particular interest for use in the in vitro diagnostic method of this invention. Exemplary industrial chemicals include the anhydrides of phthalic acid and tetrachlorophathalic acid. An exemplary pesticide is ROUNDUP® herbicide and its components, i.e., the active ingredient of which is monoisopropylamine salt of N-phosphonomethyl glycine, and a surfactant, i.e., an ethoxylated amine.

The use of the above haptens conjugated to proteins, e.g., HSA, is a unique diagnostic tool in the direct measurement of histamine released from IgE bound to basophile and mast cells. A positive reaction in this in vitro test will correlate with both clinical history and skin test reactivity. Additionally, a large number of compounds can be simultaneously tested for allergic reaction and sensitivity (i.e., ≤ 1.0 ng/ml of histamine).

A particularly desirable feature of the in vitro process of this invention is the avoidance of "false positives", i.e., spurious indication of allergic susceptibility, where such does not exist. Some methods according to the prior art suffer from this disadvantage.

## Detailed Description of the Invention

### Example 1

In this embodiment, the test procedure of the invention was used to determine allergic sensitivity of a population of exposed workers to the chemicals phthalic anhydride (PA) and tetrachlorophthalic anhydride (TCPA). The procedure was used to determine whether in vitro histamine release from basophiles challenged with human serum albumin (HSA) conjugated to PA and TCPA correlated with clinical symptoms and skin test reactivity.

Epoxy and unsaturated polyester resins are used in the manufacture of paints, plastics and electronic components. (PA) and (TCPA) are often used as cross linking agents in the manufacturing processes. The acid anhydrides have been shown to provoke asthma in workers exposed during manufacturing processes. Other studies have shown that some workers exposed to anhydrides also develop rhinitis and dermatitis. There is little information available concerning the incidence of sensitization in facilities which manufacture the acid anhydrides.

Since it is often difficult to differentiate between irritation and sensitization, data from in vitro immunological assays have been used to support a tentative diagnosis of anhydride sensitivity. The presence of specific IgE antibody in serum has been reported in sensitive patients. However, the relationship between the presence of circulating allergen-specific IgE and clinical symptoms is unclear.

Previous attempts to demonstrate the presence of TCPA specific IgE antibodies in some TCPA sensitive patients were unsuccessful. Moreover, anhydride specific IgE has been demonstrated in the serum of workers with no clinical history and/or skin test reactivity to anhydrides.

The procedure according to the present embodiment was undertaken for two purposes. First, to determine whether in vitro histamine release from test basophiles challenged with PA and TCPA conjugates correlated with clinical history and skin test reactivity to circulating anhydrides. Second, to determine the relationship between the presence of circulating specific IgE and histamine release from basophiles.

### Materials And Methods

### Tests and Control Populations

The test populations consisted of 10 workers in an anhydride manufacturing facility.

### Venipuncture:

Using multi-sample vacutainers, 20 ml of blood were drawn into sterile tubes containing disodium edetate. Plasma from the ethylene diamine tetra-acetate (EDTA) tubes was used in determination of anhydride specific IgE antibodies. The leukocytes were used in histamine assays.

### PA and TCPA

PA and TCPA flakes were obtained from the manufacturing facility. Both preparations were essentially pure. TCPA contained insoluble alkali (0.40%) and molybdenum (0.56 ppm). The PA contained ≤ 1.0 ppm of 1,4 napthoquinone.

### Preparation of PA And TCPA Human Serum Albumin (HSA) Conjugates

PA and TCPA were coupled to HSA by the method described by Ziess et al. (J. Allergy Clin. Immanol. 60: 96, (1977). Briefly, 50 mg of HSA were dissolved in 10 ml of 9% $NaHCO_3$. An equal amount of PA or TCPA was slowly added to the tube containing the HSA. After incubation at room temperature for 60 minutes, the mixture was sequentially dialyzed against 1% $NaHCO_3$ and Tris buffer containing $CaCl_2$ (1mM) and $MgCl_2$ (0.5mM). This buffer will be referred to as Tris CM. All preparations were lyophilized and stored frozen until used in the histamine release assay.

### Isolation of Leukocytes for Direct Histamine Release Assay

Following a conventional procedure, ten milliliters of blood were transferred to 15 ml plastic tubes and mixed with 2.5 ml of dextrose dextran solution. The mixture was allowed to sediment at room temperature for 60-90 minutes until a clear interface developed between the plasma and red cell layers. The leukocyte containing plasma layer was recovered and transferred to 50 ml plastic centrifuge tubes. After centrifugation at 1200 rpm for eight minutes at 4°C, the cell pellet was recovered and resuspended in 4.0 ml of TRIS-EDTA buffer. Clumps were gently broken up and 40 ml of cold TRIS-EDTA buffer was added to the tubes. Using the centrifugation conditions described above, the leukocytes were washed three times and prepared for counting. Cells were resuspended to $2 \times 10^6$ cells/ml TRIS CM buffer.

## Histamine Release Assay

The test solutions (0.25 ml) were prewarmed at 37°C in a water bath for six minutes. An equal volume of leukocytes ($2.0 \times 10^6$ cells/ml) were added to each tube. To control background fluorescence produced by test or control compounds, a second set of tubes received test or control compounds and an equal volume of Tris-CM-buffer. Background or spontaneous histamine release was ascertained by mixing cells (0.25 ml) with Tris-CM. Histamine release induced by goat anti-human IgE antibody was determined by mixing leukocytes with anti-human IgE antibody (1:500 v/v - Calbiochem). Total histamine content of leukocytes was determined after cellular lysis with 3% perchloric acid ($HClO_4$). Nonspecific histamine release was ascertained by mixing cells with N-methyl-p-methoxyphenethylamine (compound 48/80) at a concentration of $10^{-3}$ M in TRIS CM. All tubes were incubated in a 37°C water bath with frequent agitation for 60 minutes. After incubation, all tubes were centrifuged at 1600 rpm at 4°C for 15 minutes, the supernatant fluid recovered and prepared for histamine analyses. Histamine was extracted from the fluid using the automated method described by Sirjanian (Int. Arch. Allergy Appl. Immunol. 49:108, 1975 and J. Immunol. Methods 7:283, 1975). Histamine was reacted with o-phthaldehyde and the fluorescence quantified on an Alpkem histamine analyzer (Alpkem Corp. Clackamas, OR).

## Interpretation of Histamine Release Data

Histamine release values which were $\geqq$ 2.5 times the background values were considered significant.

## White Blood Cell Counts

The number of leukocytes per cc was determined by standard hemacytometer methods.

## Determination of Phthalate-Specific IgE

The TCPA-HSA and PA-HSA conjugates previously described were dissolved (1 mg/ml) in 0.05M carbonate/bicarbonate buffer, pH 9.4. The conjugates were diluted 1:10 in the buffer and pipetted (200 μl/well) to the appropriate wells of a 96-well NUNC plate. Control wells (antigen negative) received 200 μl/well of extraction buffer. The plates were covered and stored overnight at 4°C.

After allowing the plates to warm to room temperature, they were washed 4 times in a saline wash solution containing 0.1% gelatin. The serum samples were vortexed and diluted 1:5, 1:10, and 1:50 in the wash solution.

The diluted serum samples were pipetted (200 μl/well) to appropriate wells of the plate. Antibody negative control wells received 200 μl/well of wash solution. Plates were incubated for 2 hours at 37°C, washed 4 times, and 200 μl/well of rabbit anti-human IgE (Calbiochem, San Diego, CA) diluted 1:1000 in wash solution were added to all wells. Plates were incubated an additional 2 hours at 37°C, washed 4 times, and 200 μl/well of alkaline phosphatase conjugated anti-rabbit IgG diluted 1:1000 were added to all wells. The plates were incubated 2 hours at 37°C, washed 4 times, and 225 μl/well of p-nitrophenyl phosphate substrate were added to all wells. After incubating 30 minutes at room temperature, the p-nitrophenol was quantitated on a Biotek EL-310 plate reader at 405nm.

## Results

Initial studies were undertaken to optimize the reagents used in the histamine release assay. Using normal basophiles, it was determined that a 1:500 dilution of anti-human IgE would release 30-100% of the total histamine present in basophiles. Maximum non-specific histamine release by compound 48/80 was observed at a $10^{-3}$ M concentration. Anhydride-HSA conjugates initiated non-specific histamine release at high concentrations. High concentrations of PA-HSA ($10^{-1}$ M to $10^{-3}$ M) and TCPA-HSA ($10^{-1}$ M to $10^{-4}$ M) exerted a lytic effect on basophiles used in the histamine

The data in Table 1 shows the results of serum anhydride specific IgE assays and in vivo skin tests performed on the study population. The subject with known anhydride-induced asthma had no evidence of circulating IgE directed toward anhydride conjugates in the initial immunological screen. When tested eight weeks after the initial determination the serum contained minimal levels of anhydride specific IgE antibody. Circulating anhydride specific IgE was also observed in two exposed subjects. The remaining subjects had no evidence of circulating anhydride specific IgE in their sera.

The data (Table II) show that the total histamine concentrations, as well as specific (antiIgE) and non-specific histamine release (compound 48/80) varies with the test subject. Only two of the workers tested released histamine when challenged in vitro with the anhydride conjugates. Basophiles from the anhydride-sensitive asthmatic released histamine when challenged in vitro with $10^{-4}$ to $10^{-6}$ M PA-HSA and $10^{-5}$ M TCPA-HSA. Basophiles from one clinically non-sensitized worker also released histamine after in vitro challenge with a high concentration ($10^{-4}$ M) of PA-HSA. In vitro challenge with the anhydride conjugates failed to induce histamine release from the basophiles of the remaining workers.

## TABLE I

### Immunological Profile of Test Population

| | No. | Plasma PA-HSA Specific IgE | Plasma TCPA-HSA Specific IgE | Histamine Release PA-HSA | Histamine Release TCPA-HSA | Skin Test Reactivity PA-HSA | TCPA-HSA |
|---|---|---|---|---|---|---|---|
| Anhydride Sensitive Asthmatic | 1 | 1/1* | 0/1 | + | + | 1/1** | 0/1 |
| Clinically Non-sensitized Worker | 1 | 0/1 | 0/1 | + | − | 0/1 | 0/1 |
| Exposed Workers | 8 | 2/8 | 2/8 | − | − | 0/7 | 0/7*** |

\*   Negative upon initial determination.   Positive when tested eight weeks later.
\*\*   Scratch and Intradermal skin tests.
\*\*\*   One Subject unavailable for skin testing.

0 265 411

0 265 411

## TABLE II

### In Vitro Histamine Release Induced by Challenge With Anhydride-HSA Conjugates

| No. | BKG | Total Histamine | Anti IgE (1:500 v/v) | 48/80 $10^{-5}$ M | PA-HSA Conjugates | | | | TCPA-HSA Conjugates | | | |
|---|---|---|---|---|---|---|---|---|---|---|---|---|
| | | | | | $10^{-4}$ M | $10^{-5}$ M | $10^{-6}$ M | $10^{-7}$ M | $10^{-5}$ M | $10^{-6}$ M | $10^{-7}$ M | $10^{-8}$ M |
| Anhydride Sensitive Asthma (N=1) | 1.9* | 20.0 | 18.0 | 10.0 | 11.8** | 9.5** | 6.2** | 3.8 | 10.2** | 3.4 | 1.8 | 1.9 |
| Clinically Non Sensitized Worker (N=1) | 2.1 | 20.0 | 19.2 | 14.7 | 8.2** | 2.0 | 1.4 | 1.0 | 3.0 | 1.9 | 1.0 | 1.4 |
| Exposed Workers (N=8) | 1.6 ±.63 | 16.4*** ±5.9 | 10.8 ±6.5 | 13.3 ±5.2 | 2.3 ±1.1 | 2.2 ±1.0 | 2.1 ±1.0 | 2.1 ±1.0 | 2.8 ±1.6 | 2.3 ±1.0 | 2.3 ±1.0 | 2.1 ±1.0 |

\*    Results expressed as ng/ml  
\*\*   Positive histamine release assay ±2.5 times background (BKG) release.  
\*\*\*  Mean value ± standard deviation.

To determine the variability in the releasability of histamine from basophiles over a time period, the in vitro histamine assays were repeated on three workers eight weeks after the initial determination. The background and control histamine release values were similar in all subjects tested at both time intervals. The data show that the in vitro sensitivity to the anhydride conjugates increased with time when basophiles from the anhydride sensitive asthmatic were used in the assay. In the case of the anhydride-sensitive asthmatic the histamine release values increased from about 12 ng/ml to 20/ng/ml at $10^{-4}$ M PA-HSA conjugates; from about 10 to about 20 ng/ml at $10^{-5}$ M; from about 7 to about 14 ng/ml at $10^{-6}$ M from about 4.0 to about 11 ng/ml at $10^{-7}$ M. With the TCPA-HSA conjugates, histamine release values increased from about 10 ng/ml to about 14 ng/ml at about $10^{-5}$ M conjugates; from about 4 to about 11 ng/ml at $10^{-6}$ M conjugates; from about 2 to about 5 ng/ml at $10^{-7}$ M conjugates and from about 3 to about 4 ng/ml at $10^{-8}$ M conjugates.

Histamine release by basophiles from the clinically non-sensitized worker within the 8-week time period increased slightly from initial to final values within the range from about 8-9 ng/ml at $10^{-4}$ M PA-HSA conjugates, 2-3 ng/ml at $10^{-5}$ M - $10^{-7}$ M conjugates. In the case of TCPA-HSA conjugates, histamine release over the 8-week period increased slightly between initial and final values within the range of about 3-4,5 ng/ml $10^{-5}$ M conjugates and from about 2-3 ng/ml at $10^{-6}$ -$10^{-8}$ M conjugates.

The histamine release for an exposed control decreased very slightly with both the PA-HSA and TCPA-HSA conjugates from initial to final values within the range of about 1.0 - 2.0 ng/ml at $10^{-4}$ M for PA-HSA conjugate and from about 0.5 - 1.0 ng/ml at $10^{-5}$ M - $10^{-7}$ M for both conjugates (and also at $10^{-8}$ M for the TCPA-HSA conjugate).

Basophiles from the anhydride-sensitive asthmatic released histamine over a concentration range when challenged in vitro with anhydride conjugates. Hence, the data suggests that histamine release assays may be a valuable adjunct in the diagnosis of anhydride induced asthma. No false positive reactions were observed in histamine release assay when basophiles from exposed subjects were used in the assay.

Data from the histamine release assays performed at an eight week interval suggests that the anhydride-sensitive asthmatic displayed increased in vitro sensitivity over the eight week period. The reason for this phenomenon is unknown. The anhydride sensitive subject had been removed from the anhydride process area for nine months prior to testing. It is conceivable that small amounts of airborn PA liberated in the plant was sufficient to increase sensitivity. It is unlikely that the data reflects variability in the histamine release assay. When basophiles from other workers were tested in the assay at the two time intervals, there was no variation in histamine release.

The correlation between skin test reactivity and in vitro histamine release was not unexpected. The release of histamine and other mediators from mast cells and basophiles contribute to allergic signs and symptoms. In classical allergic reactions, histamine is released by an energy dependent process subsequent to the interaction of cell bound IgE antibody and allergens. Hence, in vitro histamine release would be expected to mimic in vivo reactivity.

In vitro histamine release assays using test basophiles are useful predictors of allergic sensitization. Lichtenstein et al. reported that in vitro sensitivity to ragweed predicted the in vivo response during the pollen season. J. Clin. Invest. 45:1126, 1966. Even if one assumes a fundamental dichotomy between mast cell and basophile responses to allergens, the in vitro response of the basophile may still reflect disease. Basophiles infiltrate areas of allergic inflammation and are the central cell involved in the response.

The use of in vitro histamine release assays may be advantageous when compared to skin tests. Unlike the in vivo response, in vitro histamine release is not influenced by: lipid uptake on the arachiodonic acid metabolism, levels of B-adrenergic agonists and the effects of mediators on end organs. The assay is also exquisitely sensitive. One to three ng of histamine can be detected when liberated from $2.0 \times 10^6$ cells.

The data also suggests that the in vitro assays were more sensitive when compared to in vivo skin tests. Basophiles from the anhydride sensitive asthmatic released histamine when challenged with both PA-HSA and TCPA-HSA. Skin test reactivity to TCPA was not demonstrable. The disparity in results may be due to epitope differences in reagents used in the in vivo and in vitro studies.

Skin test reactivity has been observed by other workers in several populations of anhydride sensitive asthmatics. However, a recent paper reported negative skin test to PA in asthmatic workers exposed to PA.

The data suggests that the presence of anhydride specific IgE in serum does not correlate with clinical history and skin test reactivity. Two exposed workers had demonstrable levels of anhydride specific IgE in their sera. Basophiles from both subjects failed to release histamine when challenged in vitro with anhydride conjugates. Previously, Berstein et al. have suggested that even small concentrations of serum specific IgE may be associated with symptoms. J. Allergy Clin. Immunol 69: 311,1982. It was also suggested that low level exposure to chemicals may induce sensitization and a symptomatic response in workers with specific IgE. Data from the above tests suggests that there is no relationship between the presence of circulating and cell bound IgE. Therefore, there can be no relationship between circulating IgE and the release of mediators from basophiles or mast cells. One might suggest that the presence of circulating IgE only indicates exposure and no symptomatic sensitization.

Example 2

This embodiment of the invention illustrates the use of a low molecular weight pesticidal compound as the hapten to induce histamine release from basophiles in blood plasma taken from a human subject.

Following the general procedure described in Example 1, a formulation of ROUNDUP® herbicide and major components therein, i.e., the active ingredient (monoisopropylamine salts of N-phosphonomethylglycine, common name glyphosate), surfactant (ethoxylated amine) and conjugates of the latter with HSA, were tested in vitro for direct histamine release.

The above components individually and conjugates thereof with HSA were measured for histamine release when contacted with cell-bound IgE Results of this test are shown in Table III.

## Table III

## Histamine Release Induced By Conjugates of HSA and ROUNDUP® Herbicide And Its Major Components

| | Molar Conc. | Histamine (Ng/ml) | |
|---|---|---|---|
| | | Subject | Control |
| Background | | 1.0 | 3.1 |
| Total Histamine | | 13.0 | >20.0 |
| Goat Anti-human IgE (1:500 v/v) | | 10.4 | 18.5 |
| Non-specific (48/80) | $10^{-3}$ | 8.7 | 13.5 |
| Human Serum Albumin (HSA) | $10^{-7}$ | 1.2 | 3.8 |
| Surfactant: HSA | $10^{-7}$ | 1.1 | 3.1 |
| Surfactant | $10^{-9}$ | 1.1 | 2.1 |
| Glyphosate: HSA | $10^{-5}$ | 1.1 | 2.2 |
| ROUNDUP® Herbicide | $10^{-6}$ | 1.1 | 2.5 |

Analysis of the data in Table III shows that in the direct histamine release assay, histamine released from basophiles bound to IgE in the subject's blood plasma sample when challenged with the listed haptens was less than that in the control sample. The conclusion drawn from this test is that the subject had a negative response, i.e., no indicated allergic susceptibility, to ROUNDUP® herbicide or its major components. These results correlate with skin-tests.

The protein used to couple with the low molecular weight hapten in the in-vitro tests of this invention may be any suitable naturally-occurring or synthetic protein. The only restriction on protein selection is that it must be able to couple with a low molecular weight hapten under test. Exemplary proteins include human serum albumin, the gamma globulins, hemacyanin, poly L glycines, etc.

As used herein, the term "low molecular weight haptens" refers to chemical entities having a molecular weight of less than 10,000 and preferably, less than 5,000. These haptens may in various forms, such as chemicals, especially those used in industrial and commercial operations, drugs, etc. Exemplary drugs include penicillin, ampicillin, tetracycline, aspirin, etc. The exact nature and structure of the haptens is not critical, the only requirement being a low molecular weight and having the ability to conjugate with a protein.

As low molecular weight chemical compounds are mentioned those used in industrial applications and which may cause allergic-type reactions in various subjects. The susceptibility of different people to a myriad of chemicals, e.g., perfumes, paints, varnishes, plastics, solvents, etc., makes listing of such compounds impractical. However, it is worthwhile to mention some common classes of chemicals which may be evaluated for diagnosis of allergic sensitivity thereto. Various compounds within the generic classes of α-haloacetanilides, glyphosate-type compounds, ureas, diphenyl ethers, carbamates, (thio-, di-thio, thiol), triazines, nitro-and/or trifluoromethyl phenyl-substituted anilines, amines, toluidines, etc., are amenable as co-conjugates with protein for use in the in vitro process of this invention.

Representative herbicides within the above classes include, e.g., α-chloro-2',6'-diethyl-N-methoxymethyl acetanilide (commonly known as alachlor), N-butoxymethyl- α-chloro-2',6'-diethylacetanilide (commonly known as butachlor), 2'-methyl-6'-ethyl-N-(1-methoxy-prop-2-yl)-2-chloroacetanilide (commonly known as metolachlor), 2'-t-Butyl-2-chloro-N-methoxymethyl-6'-methylacetanilide, α-chloro-N-(2-methoxy-6-methylphenyl) -N-(1-methylethoxymethyl)acetamide, α-chloro-N-(ethoxymethyl)-N-[2-methyl-6-(trifluoromethyl)phenyl]-acetamide, α-chloro-N-methyl-N-[2-methyl-6-(3-methylbutoxy) phenyl] acetamide, α-chloro-N-methyl-N-(2-methyl-6-propoxyphenyl)acetamide, N-(2-butoxy-6-methylphenyl)-α-chloro-N-methyl acetamide, isobutyl ester of (2,4-dichlorophenoxy)acetic acid, 2-chloro-N-(ethoxymethyl)-6'-ethyl-o-acetotoluidide (commonly known as acetochlor), 1-(1-cyclohexen-1-yl)-3-(2-fluorophenyl)-1-methyl urea, S-2,3,3-trichloro-allyldiisopropyl thiocarbamate (commonly known as triallate), S-2,3-dichloroallyldiisopropylthiocarbamate (commonly known as diallate), α,α,α,-trifluoro-2, 6-dinitro-N,N-dipropyl-p-toluidine (commonly known as trifluralin), N-(3,4-dichlorophenyl)-N'-methoxy-N'-methyl-urea (commonly known as linuron) 4-amino-6-tert-butyl-3-(methylthio-as-triazine-5-(4H)one (common name metribuzin), 2-chloro-4-ethylamino-6-isopropylamine-1, 3, 5-triazine (common name atrazine) and 2-[4, 5-dihydro-4-methyl-4-(1-methylethyl)-5-oxo-1H-imidazol-2-yl]-3-quinoline carboxylic acid (common name imazaquin).

Other suitable herbicides contemplated as being useful as the haptens in this invention include, by common name, the following compounds (the chemistry of each of which is readily found in standard handbooks, e.g., Pesticide Manual of the British Crop Protection Council, Fifth Edition, January, 1977, edited by H. Martin et al):

| | | |
|---|---|---|
| Ametryne | Dalapon | Methabenzthiazuron |
| Aminotriazole | Desmedipham | Methazole |
| Ammonium Sulphamate | 2,4-D | Metoxuron |
| Barban | Desmetryne | Metribuzin |
| Bentazone | Dicamba | Monolinuron |
| Benzthiazuron | Dichlorbenil | Monuron |
| Bifenox | Dichlorprop | Naptalam |
| Bromacil | Dinitramine | Neburon |
| Bromofenoxim | Dinoterb | Paraquat |
| Bromophos-Ethyl | Diquat | Picloram |
| Bromoxynil | Diuron | Propanil |
| Bromoxynil Octanoate | DSMA | Propachlor |
| Brompyrazone | Fenoprop | Propazine |
| Chloramben | Fenuron | Pyrazon |
| Chloroxuron | Flometuron | Siduron |
| Chlorthal-Dimethyl | Isoproturon | Simazine |
| Chlorthiamid | Linuron | Simetryne |
| Chlortoluron | Maleic Hydrazide | 2,4,5-T |
| Cyanazine | MCPA | MCPB |
| Cycluron | Metamitron | |

Representative insecticidal pesticides include, e.g., the following compounds (by common name): aldecarb, acephate, aldrin, aminocarb, azinphos, bendiocarb, carbaryl, chlormephos, DDT, dicofol, diflubenzuron, endothion, fenofos, fenvalerate, heptachlor, methiocarb, methomyl, methyl-and ethyl-parathion, permethrin, pyrethrin, terbufos, etc.

Representative fungicidal pesticides include the following (by common name); anilazine, benodanil, benomyl, butacarb, captafol, captan, carboxin, chloranil, chlorbromuron, chloroneb, chlorthalnil, chlorquinox, dazomet, dichlofluanid, diclone, dichloraphen, dichloran, dithianon, dodine, ferbam, folpet, mancozeb, maneb, thiabendazole, thiram, zineb, ziram, dinocat, edifenphos, Terrizole, Dowside-A, and pyrazophos.

Representative safeners (antidotes) for use with herbicides which are specifically contemplated as being suitable for use in the method of this invention include, e.g., 5-thiazolecarboxylic acid, 2-chloro-4-(trifluoromethyl),(phenylmethyl) ester, (common name "flurazole"), N-α,α-dichloroacetyl-1-oxa-4-azaspiro [4,5] decane (common name "AD-67"), N-α,α-dichloroacetyl-N,N-diallyl acetamide, N-α,α-dichloro-acetyl-2,2-dimethyl-1,3-oxazolidine, N-α, α-dichloro-acetyl-2,2,2-trimethyl-1, 3-oxazolidine, α-[(cyanomethoxy) -imino]benzenacetonitrile, α-[(1,3-dioxypyran-2-yl-methoxy)-imino] benzenacetonitrile and the like.

The basophile and/or mast cells used in the in vitro tests are obtained from any suitable mammalian source and includes blood samples from humans, laboratory animals, e.g., dogs, cats, goats, rabbits, mice, rats, guinea pigs, etc., bovine and equine animals.

While the preferred and more common mediators of histamine release arise from the cell-bound immunoglobulin (IgE) class of antibodies, the method can be used to detect homocytotropic IgG subclasses.

## Claims

1. An in vitro method for determining the susceptibility of mammalian subjects to allergic reaction from a low molecular weight hapten which comprises:

(a) Preparing a sample of a conjugate of said hapten with protein;

(b) Providing a sample of basophile or mast cells to which is bound IgE;

(c) Contacting said hapten: protein conjugate with said basophile or mast cells bound with IgE, and

(d) Measuring the amount of histamine released from said basophile and mast cells.

2. Method according to Claim 1 wherein said basophile cells are obtained from peripheral blood.

3. Method according to Claim 1 wherein said mast cells are obtained from mammalian tissue samples.

4. Method according to Claim 1 wherein said hapten has a molecular weight of less than about 10,000.

5. Method according to Claim 4 wherein said hapten has a molecular weight of less than about 5,000.

6. Method according to Claim 1 wherein said protein is HSA.

7. Method according to Claim 1 wherein said hapten is a low molecular weight chemical.

8. Method according to Claim 7 wherein said chemical is an acid anhydride.

9. Method according to Claim 7 wherein said anhydride is phthalic anhydride or tetrachlorophthalic anhydride.

10. Method according to Claim 1 wherein said hapten is a pesticide.

11. Method according to Claim 9 wherein said pesticide is ROUNDUP® herbicide.

12. Method according to Claim 10 wherein said hapten is monoisopropylamine salt of N-phosphonomethylglycine.

13. Conjugates of a low molecular weight hapten and a protein.

14. Conjugates according to Claim 13 wherein said protein is HSA.

15. Conjugates according to Claim 14 wherein said hapten is chemical.

16. Conjugates according to Claim 15 wherein said chemical is an acid anhydride.

17. Conjugates according to Claim 16 wherein said anhydride is phthalic anhydride.

18. Conjugates according to Claim 16 wherein said chemical is tetrachlorophthalic anhydride.

19. Conjugates according to Claim 14 wherein said hapten is a pesticide.

20. Conjugate according to Claim 19 wherein said pesticide is ROUNDUP® herbicide.

21. Conjugate according to Claim 19 wherein said pesticide is monoisopropylamine salt of N-phosphonomethylglycine.